(19) Europäisches Patentamt
European Patent Office
Office européen des brevets

(11) EP 0 600 013 B1

(12) **EUROPEAN PATENT SPECIFICATION**

(45) Date of publication and mention of the grant of the patent:
**19.06.1996 Bulletin 1996/25**

(21) Application number: **92918969.4**

(22) Date of filing: **18.08.1992**

(51) Int. Cl.$^6$: **A61K 7/08**, A61K 7/06, A61K 7/11

(86) International application number:
**PCT/US92/06976**

(87) International publication number:
**WO 93/03705 (04.03.1993 Gazette 1993/06)**

(54) **HAIR SPRAY COMPOSITIONS CONTAINING FLUOROSURFACTANT**

FLUORHALTIGE OBERFLÄCHENAKTIVE SUBSTANZEN ENTHALTENDE HAARSPRAY-ZUSAMMENSETZUNGEN

COMPOSITIONS D'ATOMISATION POUR CHEVEUX CONTENANT UN FLUOROTENSIOACTIF

(84) Designated Contracting States:
**AT BE CH DE DK ES FR GB GR IT LI LU NL SE**

(30) Priority: **19.08.1991 US 747163**
**15.05.1992 US 883973**

(43) Date of publication of application:
**08.06.1994 Bulletin 1994/23**

(73) Proprietor: **THE PROCTER & GAMBLE COMPANY**
**Cincinnati, Ohio 45202 (US)**

(72) Inventor: **PEFFLY, Marjorie, Mossman**
**Cincinnati, OH 45249 (US)**

(74) Representative: **Brooks, Maxim Courtney**
**Procter & Gamble Limited**
**Whitley Road**
**Longbenton**
**Newcastle-upon-Tyne NE12 9TS (GB)**

(56) References cited:

| | |
|---|---|
| EP-A- 0 412 704 | EP-A- 0 412 707 |
| FR-A- 2 450 105 | GB-A- 1 598 567 |
| US-A- 3 993 744 | US-A- 3 993 745 |

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 600 013 B1

## Description

The present invention relates to hair spray compositions which hold hair in place and are easily and uniformly dispersed from aerosol or pump spray dispensers.

The desire to have the hair retain a particular shape is widely held. The most common methodology for accomplishing this is the application of a composition to dampened hair, after shampooing and/or conditioning, or to dry, styled hair. These compositions provide temporary setting benefits and they can be removed by water or by shampooing. The materials used in the compositions to provide the setting benefits have generally been resins and have been applied in the form of mousses, gels, lotions or sprays.

Many people desire a high level of style retention, or hold, from a hair spray composition. In typical hair sprays, hold is achieved by the use of resins, such as AMPHOMER® (RTM), supplied by National Starch and Chemical Company, and GANTREZ SP 225® (RTM), supplied by GAF. These resins generally have a weight average molecular weight of from about 40,000 to about 150,000. When such resins are incorporated into pump and aerosol hair sprays, they can provide hold and good sprayability. In general, as hair hold for hair spray compositions is increased, the tactile feel of the hair becomes stiffer and dense, less desirable. It is desirable to provide hair spray products which could provide an improved combination of hair hold and hair feel characteristics.

It has now been found that the use of higher weight average molecular weight resins can be beneficial due to the increase in style retention generally expected to be provided by such resins and the general decrease in the amount of resin required for incorporation to achieve good style retention.

Unfortunately, the use of such higher molecular weight resins, such as those having a weight average molecular weight of greater than about 300,000, in aerosol and pump spray formulations has been difficult. Such formulations tend to exhibit poor spray quality. For example, they tend to have spray patterns characterized by wet drippy centers or to have spray characterized by streaming rather than a fine misting of hair spray particles. Since the difficulties in spraying higher molecular weight resins leads to practical concerns, such as clogging and poor application consistency, and also typically lead to poor hair feel and hold, it is an object of this invention to provide hairspray compositions which provide the benefits believed to be obtainable for high molecular weight resins without incurring the performance negatives that result from the difficulties in spraying such compositions.

Hair sprays have also been conventionally formulated with high amounts of monohydric alcohol solvents, such as ethanol and isopropanol, and relatively low amounts of water since the presence of water adversely affects spray quality. However, it is now particularly desirable to formulate hair spray compositions with reduced levels of volatile organic solvents, such as ethanol and, isopropanol. One way is to increase the levels of water in the formulations. In doing so, it would be highly desirable to provide reformulated products which overcome the problems conventionally associated with the addition of water to hair spray products. In particular, higher levels of water can impart increased drying time and negatively impact hair feel.

It can be particularly difficult to formulate silicone macromer-containing hair setting resins into hair care compositions. Silicone macromer-containing polymers can be used to make hair spray compositions which combine hair styling (alternately, hair setting) with improved hair feel, e.g., softness relative to conventional hair styling polymers. These silicone macromer-containing polymers can be utilized at a wide variety of molecular weights. The lower molecular weight polymers, such as those from about 50,000 to 300,000 tend to be characterized by poor spray quality relative to conventional hair setting polymers. The higher molecular weights become difficult to formulate into compositions with good spray quality. The molecular weight of the resin used in these types of compositions is relatively high in relation to the resins most commercial hair sprays, thus exacerbating the spray quality problems.

Fluorinated surfactants, herein referred to as fluorosurfactants, are known for use in shampoo and rinse compositions. US-A-4,183,367, Goebel et al., issued January 15, 1980, discloses the use of fluorinated cationic and amphoteric surfactants in shampoo and rinse compositions in order to promote more rapid drying of washed hair by reducing the amount of water remaining in the hair. GB-A-1,599,414, Callingham et al., published September 30, 1981, discloses the use of anionic, amphoteric, nonionic or cationic fluorosurfactants in shampoo compositions to obtain anti-grease effects.

Fluorosurfactants have been disclosed for use in hair spray compositions containing conventional hair setting resins and conventional, low levels of water by US-A-3,993,745, Cella et al., issued November 23, 1976, and US-A-3,993,744, Cella et al., issued November 23, 1976. It is disclosed that treatment of the hair with these compositions effectively retards the excess flow of sebum.

It has now been discovered that the addition of an ionic fluorosurfactant to hair spray solutions containing an ionic resin and relatively high levels of water can provide hair spray compositions with excellent performance. Such compositions can further provide improved hair feel with zero or relatively little reduction in hair hold compared with conventional, low water content compositions. It has also been found that the combination of fluorosurfactants with silicone macromer-containing hair styling resins, in general, can provide excellent hair spray performance for both low and particularly for high water content hair spray compositions. The use of fluorosurfactants in hair sprays containing particularly high molecular weight containing ionic hair setting resins (e.g. about 300,000 and higher) is especially advantageous in that such

high molecular weight resins are particularly difficult to formulate into sprayable compositions with good spray characteristics.

These and other benefits as may be apparent from the description below can be obtained by the present invention.

The present invention relates to low volatile organic solvent hair spray products comprising a hairspray composition and a spray dispenser means for containing and spraying said hairspray composition, said hairspray composition being contained in said spray dispenser means, wherein said hairspray composition characterized in that it comprises:

(a) from 0.01% to 2%, by weight, of an ionic fluorosurfactant;
(b) from 0.1% to 15%, by weight, of an anionic and/or amphoteric hair setting resin; and
(c) a liquid vehicle comprising from 10% to 70%, by weight of the composition, water and from 30% to 80%, by weight of the composition, of monohydric alcohol.

Unless otherwise indicated, all percentages herein are by weight of the hair spray composition.

The essential, as well as optional, components used in the present invention are described below.

The hairspray products the present invention contain, as an essential component, an ionic fluorosurfactant. The ionic fluorosurfactant can be selected from the group consisting of cationic fluorosurfactants, anionic fluorosurfactants, amphoteric fluorosurfactants, zwitterionic fluorosurfactants, and mixtures thereof.

The fluorosurfactant is essential for obtaining good spray-ability. It is believed that this benefit is provided by an interaction which causes viscosity reduction of the hair spray compositions.

A fluorosurfactant is a surface active agent. Commonly, a fluorosurfactant is described as a molecule consisting of a hydrophilic moiety and a hydrophobic moiety containing a fluorine substituted hydrocarbon. Fluorosurfactants useful in the present compositions can be linear or branched alkyl, alkenyl or alkylaryl fluorohydrocarbons having a chain length of preferably 3 to 18 carbon atoms and being fully or partially fluorinated. The hydrophilic moiety can be sulfate, phosphate, phosphonate, sulfonate, amine, amine salts, quaternary ammonium, carboxylate, and any combination thereof. Also, there can be a bridging moiety between the hydrophilic and hydrophobic moieties, such as an amido alkylene group for example.

Fluorosurfactants useful in the hairspray products of the present invention are any fluorinated surfactants having an ionic character. Such useful fluorosurfactants can be any fluorinated hydrocarbon surfactants having the requisite character.

A subset of the ionic fluorosurfactants useful in the present compositions are perfluorinated compounds which can be represented by the formula

$$CF_3\text{-}(CF_2)_x\text{-}(CH_2)_y\text{-}Z$$

where Z is a water solubilizing group of either organic or inorganic character, x is an integer which is generally from 2 to 17, particularly from 7 to 11, and y is an integer from 0 to 4, and said compounds may be cationic, anionic, amphoteric or zwitterionic, depending upon the nature of the grouping or groupings encompassed by Z. The Z groups may be or may comprise sulfate, sulfonate, carboxylate, amine salt, quaternary ammonium, phosphate, phosphonate, and combinations thereof. The perfluorinated compounds are known in the art. These compounds are described in US-A-4,176,176, Cella et al., issued November 27, 1979; US-A-3,993,745, Cella et al., issued November 23, 1976, and US-A-3,993,744, Cella et al., issued November 23, 1976.

Suitable anionic fluorosurfactants can have anionic moieties which include carboxylates, sulfates, sulfonates, phosphonates and phosphates or any combination thereof. Counterions therefore can include sodium, $NH_4$, magnesium, potassium, tri-ethanolamine, di-ethanolamine, and similar moieties. Suitable cationic fluorosurfactants can have cationic moieties which include quaternary ammonium compounds where the counterions can be chloride or any other halide, methosulfate, ethosulfate, phosphate, acetate, and other similar moieties. Also, suitable cationic fluorosurfactants can have cationic moieties which include primary, secondary and tertiary amine salts of acids such as hydrochloric, lactic, phosphoric, sulfuric and other similar acids. Amphoteric fluorosurfactants contain both a carboxylate and an amine group. Zwitterionic fluorosurfactants contain an anionic moiety such as a carboxylate, sulfate, sulfonate, and phosphate group or other similar groups as well as a cation moiety such as a quaternary ammonium or amine salt. It should be noted that the terms "amphoteric" and "zwitterionic" have been used interchangeably by chemical supply companies and that the classification of fluorosurfactants herein may differ from that given by supplying companies.

Cationic fluorosurfactants preferred for use in the present hairspray products include fluorinated alkyl quaternary ammonium salts having a variety of anionic counter ions, including iodide, chloride, methosulfate, phosphate, and nitrate salts, preferably an iodide; and those fluorosurfactants conforming to the formula $R_fCH_2CH_2SCH_2CH_2N^+(CH_3)_3[CH_3SO_4]^-$ wherein $R_f=F(CF_2CF_2)_{3-8}$, such as Zonyl FSC® (RTM) supplied by DuPont. Preferred fluorinated alkyl quaternary ammonium iodides are supplied under the tradename Fluorad FC-135® (RTM) supplied by 3M.

Anionic fluorosurfactants preferred for use in the present hairspray products are mono-, and bis-perfluoroalkyl phosphates, such as Zonyl FSP® (RTM) supplied by DuPont and conforming to the general formulae $(R_fCH_2CH_2O)P(O)(ONH_4)_2(R_fCH_2CH_2O)_2P(O)(ONH_4)$ wherein $R_f=F(CF_2CF_2)_{3-8}$; mono- and bis-fluoroalkyl phosphates, having a variety of cationic counterions such as ammonium, sodium, potassium, triethanolamine and diethanolamine salts, preferably ammonium salts, complexed with non-fluorinated quats, preferably aliphatic quaternary methosulfates, such as Zonyl FSJ® (RTM) supplied by DuPont; perfluoroalkyl sulfonic acid having a variety of cationic counterions such as ammonium, sodium, potassium, triethanolamine and diethanolamine salts, preferably ammonium salts, such as Zonyl TBS® (RTM) supplied by DuPont and conforming to the formula $R_fCH_2CH_2SO_3X$ wherein $R_f=F(CF_2CF_2)_{3-8}$ and X=H and $NH_4$; telomer phosphates, having a variety of cationic counterions such as ammonium, sodium, potassium, triethanolamine and diethanolamine salts, preferably diethanolamine salts, such as Zonyl RP® (RTM) supplied by DuPont; amine perfluoroalkyl sulfonates, such as Fluorad FC-99® (RTM) supplied by 3M; ammonium perfluoroalkyl sulfonates, such as Fluorad FC-93® (RTM), Fluorad FC-120® (RTM) and L-12402® (RTM), supplied by 3M; potassium perfluoroalkyl sulfonates, such as Fluorad FC-95® (RTM) and Fluorad FC-98® (RTM) supplied by 3M; potassium fluorinated alkyl carboxylates, such as Fluorad FC-129® (RTM) and supplied by 3M; ammonium perfluoroalkyl carboxylates, such as Fluorad FC-143® (RTM) supplied by 3M; and those fluorosurfactants conforming to the general formula $R_fCH_2CH_2SCH_2CH_2CO_2Li$ wherein $R_f=F(CF_2CF_2)_{3-8}$, such as Zonyl FSA® supplied by DuPont.

Preferred anionic fluorosurfactants are mixed mono- and bis-perfluoroalkyl phosphates, ammonium salts; mixed mono- and bis-fluoroalkyl phosphate, ammonium salts, complexed with aliphatic quaternary methosulfates; perfluoroalkyl sulfonic acid, ammonium salts; mixed telomer phosphate diethanolamine salts; amine perfluoroalkyl sulfonates; ammonium perfluoroalkyl sulfonates; potassium perfluoroalkyl sulfonates; potassium fluorinated alkyl carboxylates; ammonium perfluoroalkyl sulfonates; and ammonium perfluoroalkyl carboxylates.

Amphoteric fluorosurfactants preferred for use in the present hairspray products are fluorinated alkyl amphoterics such as Fluorad FC-100® (RTM) supplied by 3M; and the experimental amphoteric fluorosurfactant L-12231 supplied by 3M.

Zwitterionic fluorosurfactants preferred for use in the present by weight are those fluorosurfactants conforming to the formula $R_fCH_2CH(OCOCH_3)CH_2N^+(CH_3)_2CH_2CO_2^-$ wherein $R_f=F(CF_2CF_2)_{3-8}$ such as Zonyl FSK® (RTM) supplied by DuPont.

Preferably, mixtures of amphoteric or zwitterionic and anionic fluorosurfactants and mixtures of anionic and cationic fluorosurfactants can be used in the present hair spray products. Preferred zwitterionic and anionic mixtures for use are a mixture of Zonyl FSK® (RTM) supplied by by DuPont and Fluorad FC-100® (RTM) supplied by 3M and a mixture of Zonyl FSK® (RTM) supplied by DuPont, and Zonyl TBS® (RTM) supplied by DuPont. Preferred mixtures of anionic and cationic fluorosurfactants are a mixture of potassium perfluoroalkyl sulfonates, supplied as Fluorad FC-95® (RTM) by 3M and fluorinated alkyl quaternary ammonium iodides, supplied as Fluorad FC-135® (RTM) supplied by 3M.

It may be beneficial to enhance the solubility of a less soluble fluorosurfactant by combining it with a more soluble fluorosurfactant. For example, mixing Zonyl FSK® (RTM) with the more soluble Fluorad FC-100® (RTM) aids the solubility of the less soluble Zonyl FSK® (RTM).

An ionic fluorosurfactant, preferably selected from the group consisting of cationic fluorosurfactants, anionic fluorosurfactants, amphoteric fluorosurfactants, zwitterionic fluorosurfactants and mixtures thereof, is used in the hairspray products of the present invention at levels of from 0.01% to 2%, preferably from 0.01% to 1.5%. Most preferred is a level of from 0.02% to 1% by weight.

An ionic resin useful for styling hair is an essential component of the present invention. The resin is essential for providing hair hold.

As used herein, "ionic resin" means any ionic polymer or polymers, natural or synthetic, that can provide hair setting benefits. Polymers of this type are well known in the art. Generally, the level of hair styling polymer used will be at least 0.1%, by weight, of the composition. Typically, it will be present at a level of from 0.1% to 15%, preferably from 0.5% to 8%, more preferably from 1% to 5% by weight.

The polymers hereof can have a weight average molecular weight of any level that is useful for providing hair setting benefits. Generally the weight average molecular weight will be at least about 30,000, typically at least about 50,000, preferably at least about 60,000. It is not intended for there to be any specific, critical upper limit for molecular weight, except that the resulting composition, with the aid of fluorosurfactant, must be sprayable. Generally, the molecular weight will be less than about 10,000,000, preferably less than 5,000,000, more preferably less than about 3,000,000.

The silicone macromer-containing hair setting resins hereof preferably have a weight average molecular weight of at least about 50,000, more preferably at least about 70,000, 100,000, even preferably from about 100,000, to about 1,000,000, most preferably from about 125,000 to about 200,000.

The resins hereof also are of ionic character. As used herein, "ionic resin", "ionic polymer", or the term "ionic character" in reference to polymers or monomers of which such polymers are comprised, means polymers that are anionic, cationic, amphoteric, zwitterionic, or otherwise can exist in the liquid vehicle of the hair styling composition in dissociated form.

Any type of ionic polymer which is soluble or dispersible in the liquid carrier can be used in the present invention. A wide variety of such types of polymers are known in the art.

The ionic polymers hereof can be homopolymers, copolymers, terpolymers, etc. As used herein, the term "polymer" shall encompass all of such types of polymeric materials.

As an essential aspect, the resins hereof must comprise monomers of an ionic character. For convenience in describing the polymers hereof, monomeric units present in the polymers may be referred to as the monomers from which they can be derived. The ionic monomers can be derived from polymerizable ionic starting monomers, or from polymerizable nonionic monomers which are modified subsequent to polymerization to be of ionic character. Ionic monomers hereof include salts, acids and bases of monomers exemplified herein.

Examples of anionic monomers include:

(i) unsaturated carboxylic acid monomers such as acrylic acid, methacrylic acid, maleic acid, maleic acid half ester, itaconic acid, fumaric acid, and crotonic acid;
(ii) half esters of an unsaturated polybasic acid anhydride such as succinic anhydride, phthalic anhydride or the like reacted with a hydroxyl group-containing acrylate and/or methacrylate such as hydroxyethyl acrylate, hydroxyethyl methacrylate, hydroxypropyl acrylate, and the like;
(iii) monomers having a sulfonic acid group such as styrenesulfonic acid, sulfoethyl acrylate and methacrylate, and the like; and
(iv) monomers having a phosphoric acid group such as acid phosphooxyethyl acrylate and methacrylate, 3-chloro-2-acid phosphooxypropyl acrylate and methacrylate, and the like.

Examples of the amphoteric monomers include zwitterionized derivatives of the aforementioned amine derivatives of (meth)acrylic acids or the amine derivatives of (meth)acrylamide such as dimethylaminoethyl (meth)acrylate, dimethylaminopropyl(meth)acrylamide by a halogenated fatty acid salt such as potassium monochloroacetate, sodium monobromopropionate, aminomethylpropanol salt of monochloroacetic acid, triethanolamine salts of monochloroacetic acid and the like; and amine derivatives of (meth)acrylic acid or (meth)acrylamide, as discussed above, modified with propanesultone.

These amphoteric monomers, like the aforementioned cationic monomers, can be polymerized in amphoteric form or, as an alternative, they can also be polymerized in the form of their precursors, which are then converted into the amphoteric state.

Preferred ionic monomers include acrylic acid, methacrylic acid, dimethylaminoethyl methacrylate, quaternized dimethylaminoethyl methacrylate, maleic acid, maleic anhydride half esters, crotonic acid, itaconic acid, diallyldimethyl ammonium chloride, polar vinyl heterocyclics such as vinyl imidazole, vinyl pyridine, styrene sulfonate, and mixtures thereof. Especially preferred ionic monomers include acrylic acid, dimethylaminoethyl methacrylate, quaternized dimethylaminoethyl methacrylate, salts of amines and acids listed above, and mixtures thereof.

The polymers hereof should contain at least 1%, by weight, ionic monomer, preferably at least 2%, more preferably at least 5%.

The resins hereof can also contain nonionic monomers including, both high polarity monomers and low polarity monomers.

The ionic resins hereof will generally comprise from 1% to 100% ionic monomers and from 0% to 99% nonionic monomers, preferably from 2% to 75% ionic monomers and from 25% to 98% nonionic monomers, more preferably from 5% to 50% ionic monomers and from 50% to 95% nonionic monomers.

Representative examples of nonionic monomers are acrylic or methacrylic acid esters of $C_1$-$C_{24}$ alcohols, such as methanol, ethanol, 1-propanol, 2-propanol, 1-butanol, 2-methyl-1-propanol, 1-pentanol, 2-pentanol, 3-pentanol, 2-methyl-1-butanol, 1-methyl-1-butanol, 3-methyl-1-butanol, 1-methyl-1-pentanol, 2-methyl-1-pentanol, 3-methyl-1-pentanol, t-butanol, cyclohexanol, 2-ethyl-1-butanol, 3-heptanol, benzyl alcohol, 2-octanol, 6-methyl-1-heptanol, 2-ethyl-1-hexanol, 3,5-dimethyl-1-hexanol, 3,5,5-trimethyl-1-hexanol, 1-decanol, 1-dodecanol, 1-hexadecanol, 1-octadecanol, and the like, the alcohols having from about 1-24 carbon atoms with the average number of carbon atoms preferably being from about 4-18, more preferably from about 4-12; styrene; chlorostyrene; vinyl esters such as vinyl acetate; vinyl chloride; vinylidene chloride; acrylonitrile; alpha-methylstyrene; t-butylstyrene; butadiene; cyclohexadiene; ethylene; propylene; vinyl toluene; alkoxyalkyl (meth)acrylate, such as methoxy ethyl (meth)acrylate and butoxyethyl (meth)acrylate; and mixtures thereof. Other nonionic monomers include acrylate and methacrylate derivatives such as allyl acrylate and methacrylate, cyclohexyl acrylate and methacrylate, and methacrylate, oleyl acrylate and methacrylate, benzyl acrylate and methacrylate, tetrahydrofurfuryl acrylate and methacrylate, ethylene glycol di-acrylate and -methacrylate, 1,3-butyleneglycol di-acrylate and -methacrylate, diacetonacrylamide, isobornyl (meth)acrylate, and the like.

Preferred nonionic monomers include n-butyl methacrylate, isobutyl methacrylate, 2-ethylhexyl methacrylate, methyl methacrylate, t-butylacrylate, t-butylmethacrylate, and mixtures thereof.

Representative polar nonionic monomers include acrylamide, N,N-dimethylacrylamide, methacrylamide, N-t-butyl acrylamide, methacrylonitrile, acrylamide, acrylate alcohols (e.g. $C_2$-$C_6$ acrylate alcohols such as hydroxyethyl acrylate,

hydroxypropyl acrylate), hydroxyethyl methacrylate, hydroxypropyl methacrylate, vinyl pyrrolidone, vinyl ethers, such as methyl vinyl ether, acyl lactones and vinyl pyridine, allyl alcohols, vinyl alcohols and vinyl caprolactam.

Examples of anionic hair spray resins are copolymers of vinyl acetate and crotonic acid, terpolymers of vinyl acetate, crotonic acid and a vinyl ester of an alpha-branched saturated aliphatic monocarboxylic acid such as vinyl neodecanoate; and copolymers of methyl vinyl ether and maleic anhydride (molar ratio about 1:1) wherein such copolymers are 50% esterified with a saturated aliphatic alcohol containing from 1 to 4 carbon atoms such as ethanol or butanol; and acrylic copolymers and terpolymers containing acrylic acid or methacrylic acid as the anionic radical containing moiety such as copolymers with methacrylic acid, butyl acrylate, ethyl methacrylate, etc. Another example of an acrylic polymer which can be employed in the compositions of the present invention is a polymer of tertiary-butyl acrylamide, acrylic acid, and ethyl acrylate.

An example of an amphoteric resin which can be used in the present invention is Octylacrylamide/Acrylates/Butylaminoethyl Methacrylate Copolymer, described generally in US-A-4,192,861 as being a polymer of N-tert-octyl acrylamide, methyl methacrylate, hydroxypropyl methacrylate, acrylic acid and t-butyl aminoethyl methacrylate, of appropriate molecular weight for purposes hereof.

Still other organic, ionic resins include carboxymethyl cellulose, copolymers of PVA and crotonic acid, copolymers of PVA and maleic anhydride, sodium polystyrene sulfonate, PVP/ethylmethacrylate/methacrylic acid terpolymer, vinyl acetate/crotonic acid/vinyl neodecanoate copolymer, octylacrylamide/acrylates copolymer, monoethyl ester of poly(methyl vinyl ether-maleic acid), and octylacrylamide/acrylate/butylaminoethyl methacrylate copolymers. Mixtures of polymers may also be used.

Preferred ionic resins are silicone-containing ionic polymers. Silicone-containing ionic polymers are described, for example, in: EP-A-0 408 311 A2 Hayama, et al., US-A-5,061,481, issued October 29, 1991, Suzuki et al., US-A-5,106,609, Bolich et al., issued April 21, 1992, US-A-5,100,658, Bolich et al., issued March 31, 1992, US-A-5,100,657, Ansher-Jackson, et al., issued March 31, 1992, US-A-5,104,646, Bolich et al., issued April 14, 1992, U.S. Serial No. 07/758,319, Bolich et al, filed August 27, 1991, and U.S. Serial No. 07/758,320, Torgerson et al., filed August 27, 1991.

Preferred silicone-containing ionic polymers contain an organic polymeric backbone, preferably a vinyl backbone, having a Tg or a Tm above about -20°C and, grafted to the backbone, a siloxane macromer having a weight average molecular weight of preferably at least about 500, more preferably from about 1,000 to about 100,000, even more preferably from about 2,000 to about 50,000, most preferably about 5,000 to about 20,000. In addition to the graft copolymers described above, silicone-containing polymers also include block copolymers preferably containing up to about 50% (more preferably from about 10% to about 40%) by weight of one or more siloxane blocks and one or more non-silicone blocks (such as acrylates or vinyls).

The silicone-containing ionic polymers preferred for use herein are such that when formulated into the finished hair care composition, and dried, the polymer phase separates into a discontinuous phase which includes the silicone portion and a continuous phase which includes the organic portion.

The silicone-containing ionic polymers generally comprise nonionic silicone-containing monomers together with ionic monomers as described above, and can also contain non-silicone-containing nonionic monomers, also described above. The silicone-containing monomers also can be ionically charged and, as such, contribute, in part or in whole, to the overall charge density of the polymer.

Examples of useful silicone-containing ionic polymers and how they are made are described in detail in US-A-4,693,935, Mazurek, issued September 15, 1987, and US-A-4,728,571, Clemens et al., issued March 1, 1988.

The silicone-containing ionic polymers hereof will generally comprise about 0.01% to about 50% of silicone-containing monomer, preferably from about 0.5% to about 40% of ionic monomer, more preferably from about 2% to about 25%.

The silicone-containing monomer has the general formula:

$$X(Y)_nSi(R)_{3-m}Z_m$$

wherein X is a vinyl group copolymerizable with the other monomers of the polymer; Y is a divalent linking group; R is a hydroxyl, lower alkyl (eg. $C_1$-$C_4$), aryl, alkylamino, alkaryl, hydrogen or alkoxy; Z is a monovalent siloxane polymeric moiety having a number average molecular weight of at least about 500, and is pendant from the organic polymeric backbone, described above; n is 0 or 1; and m is an integer from 1 to 3. Of course, Z should be essentially unreactive under polymerization conditions. The silicone-containing monomer preferably has a weight average molecular weight of at least about 1,000, preferably from about 1,000 to about 100,000, more preferably from about 2,000 to about 50,000, most preferably from about 5,000 to about 20,000. Preferably, it is of the formula:

$$X-\overset{O}{\overset{\|}{C}}-O-(CH_2)_q-(O)_p-Si(R^1)_{3-m}\ Z_m$$

wherein m is 1, 2 or 3 (preferably m = 1); p is 0 or 1; R is alkyl or hydrogen; q is an integer from 2 to 6; X is

$$\underset{R^2}{\underset{|}{CH}}=\underset{R^3}{\underset{|}{C}}-\ ;$$

$R^2$ is hydrogen or -COOH (preferably $R^2$ is hydrogen); $R^3$ is hydrogen, methyl or -$CH_2COOH$ (preferably $R^3$ is methyl); Z is

$$R^4-(-\underset{R^6}{\underset{|}{\overset{R^5}{\overset{|}{Si}}}}-O-)_r;$$

$R^4$, $R^5$, $R^6$ independently are alkyl, alkoxy, alkylamino, aryl, alkaryl, hydrogen, or hydroxyl (preferably alkyl, more preferably methyl); and r is an integer of at least about 5, preferably from about 10 to about 1500, more preferably from about 70 to about 700 , most preferably from about 100 to about 250.

The silicone-containing monomers of the ionic polymers hereof can be polymerized in a silicone-containing monomer form. Alternatively, they can be polymerized in the form of their non-silicone containing precursor, and a silicone group can then be added. For example, carboxylate-containing monomers, such as acrylic acid, can be polymerized and then reacted with a silicone-containing compound with a terminal epoxy group. The result will, in general, be a silicone-containing monomer in the polymer having an equivalent structure to the formula $X(Y)_nSi(R)_{3-m}Zm$, described above, and is intended to be encompassed herein.

The preferred silicone-containing ionic polymers useful in the present invention generally comprise from 0% to about 98% (preferably from about 5% to about 98%, more preferably from about 50% to about 90%) of nonionic monomer, from 1% to about 98% (preferably from about 15% to about 80%) of ionic monomer, with from about 0.1% to about 50% (preferably from about 0.5% to about 40%, most preferably from about 2% to about 25%) of the monomers being silicone-containing monomer. The combination of the non-silicone-containing monomers preferably is from about 50% to about 99% (more preferably about 60% to about 99%, most preferably from about 75% to about 95%) of the polymer.

Exemplary silicone-containing ionic polymers for use in the present invention include the following:

(i) acrylic acid/n-butylmethacrylate/polydimethylsiloxane (PDMS) macromer-20,000 molecular weight
(ii) dimethylaminoethyl methacrylate/isobutyl methacrylate/2-ethylhexyl-methacrylate/PDMS macromer-20,000 molecular weight
(iii)t-butylacrylate/acrylic acid/PDMS macromer-10,000 molecular weight
(iv)t-butylacrylate/acrylic acid/PDMS macromer-10,000 molecular weight

As is known in the art, polymers which have acidic functionalities, such as carboxyl groups, are usually used in at least partially neutralized form to promote solubility/dispersibility of the polymer. In addition, use of the neutralized form aids in the ability of the hair spray compositions to be removed from the hair by shampooing. In general, it is preferred that from about 10% to 100%, more preferably from about 20% to about 90%, even more preferably from about 40% to about 85%, of the acidic monomers of the polymer be neutralized.

Any conventionally used base, organic or metallic, may be used for neutralization of the polymers. Metallic bases are particularly useful in the present compositions. Hydroxides, where the cation is an alkali metal or an alkaline earth metal, are suitable neutralizers for use in the present hair spray products.

Preferred neutralizing agents for use in the hairspray products of the present invention are potassium hydroxide and sodium hydroxide.

Examples of other suitable neturalizing agents which may be included in the hair spray products of the present invention include amines, especially amino alcohols such as 2-amino-2-methyl-1,3-propanediol (AMPD), 2-amine-2-ethyl-1,3-propanediol (AEPD), 2-amino-2-methyl-1-propanol (AMP), 2-amino-1-butanol (AB), monoethanolamine

(MEA), diethanolamine (DEA), triethanolamine (TEA), monoisopropanolamine (MIPA), diisopropanol-amine (DIPA), tri-isopropanolamine (TIPA) and dimethyl steramine (DMS). Particularly useful neutralizing agents are mixtures of amines and metallic bases.

Polymers having basic functionalities, e.g., amino groups, are preferably at least partially neutralized with an acid, e.g., hydrogen chloride.

In general, in the hair spray products hereof, and cationic fluorosurfactants preferably will not be used in significant amounts in combination with anionic resins.

The hair spray products of the present invention also include a liquid vehicle. This can comprise any of those conventionally used in resin hair spray formulations. 98% by weight.

Organic solvents suitable for use in the liquid vehicle of the present products are $C_1$-$C_6$ alkanols, carbitol, acetone and mixtures thereof. $C_1$-$C_6$ alkanols preferred for use in the present compositions are $C_2$-$C_4$ monohydric alcohols such as ethanol, isopropanol and mixtures thereof. Water is also a preferred solvent for use in the liquid vehicle of the present hair spray compositions.

Preferably, the liquid vehicle for the present products is selected from the group consisting of $C_1$-$C_6$ alkanols, water, carbitol, acetone and mixtures thereof. More preferably, the liquid vehicle of the present composition is selected from the group consisting of water and $C_2$-$C_4$ monohydric alcohols such as ethanol and isopropanol, and mixtures thereof.

In general, the liquid vehicle is a mixture of water and organic solvents.

In general, benefits of the present invention due to use of the fluorosurfactant in the products described herein is improved sprayability, improved spray-performance, and improved hair feel at a constant level of hold.

In one aspect of the invention, the hair setting resin is of particularly high weight average molecular weight, i.e. weight average molecular weight above about 300,000, especially above about 500,000. It has been found that surprisingly effective hair spray performance can be obtained using these high molecular weight resins in combination with fluorosurfactants, and that the fluorosurfactants are especially effective at providing the resins with characteristics in hair spray products such that they can be sprayed with good spray quality, especially with respect to silicone macromer-containing hair setting resins, which are preferably used at higher molecular weights that have been typically used for conventional hair setting resins. This is important since such high molecular weight resins are normally difficult to formulate into products that can be applied via a spray application with good spray quality.

In another aspect of the invention, what is provided is a hair spray product which comprises a silicone macromer-containing ionic hair setting resin, an ionic fluorosurfactant, and a liquid vehicle. The weight average molecular weight of the silicone macromer-containing hair setting resins can be of any level suitable for providing effective hair styling. Typically, it will be at least about 50,000, more typically at least about 70,000, more preferably at least about 100,000. Even at relatively low molecular weights, the use of fluorosurfactant can improve hair feel while imparting zero or a relatively minor loss in hair styling or hair hold performance. Furthermore, at higher molecular weights such as those above about 175,000, particularly those of about 200,000 and higher, improved hair feel relative to hair hold performance as well as improved spray quality can be obtained.

In general, the molecular weight for the upper end of the resins herein is limited only by practical concerns with respect to formulating suitable compositions. Typically molecular weight (wt. average) will be no more than about 10,000,000, more typically no more than about 3,000,000, preferably no more than about 1,000,000.

This invention provides hair spray products comprising hair spray compositions, which contain reduced levels of volatile organic solvents, provided within a means for containing and spraying the composition. A reduced volatile organic solvent hair spray composition of the present invention comprises hair setting resin fluorosurfactant, and no more than 80% volatile organic solvents (which include, for purposes hereof, volatile silicone fluids and excludes water). As used herein, volatile organic solvents means solvents which have at least one carbon atom and exhibit a vapor pressure of greater than 0.1 mm Hg at 20°C.

In the reduced volatile organic solvent hair spray products hereof, the hair spray compositions comprise at least 10%, by weight, of water. It is also specifically contemplated that they may contain at least about 11%, 12%, 13%, 14%, 15% by weight, or more water. The reduced volatile organic solvent compositions hereof will comprise up to 90% by weight, preferably up to 70%, more preferably up to 60%, even more preferably no more than 50% by weight, water; and from 10% to 80% by weight, preferably from 20% to 80%, more preferably from 40% to 80% by weight, of volatile organic solvent. It is also contemplated that the compositions can be limited to containing no more than 75%, 65%, 55% by weight, or other levels of volatile organic solvents. Hair spray compositions containing relatively high levels of water, in combination with the volatile organic solvents can surprisingly exhibit improved hair spray performance, particularly improved hair feel for a constant level of hair hold, when fluorosurfactant is used compared to conventional commercial hair spray compositions.

A preferred reduced volatile organic solvent hair spray composition of the present invention contains from 0.01% to 2% by weight of a fluorosurfactant preferably selected from the group consisting of cationic fluorosurfactants, anionic fluorosurfactants, amphoteric fluorosurfactants, zwitterionic fluorosurfactants and mixtures thereof; from 0.1% to 15% by weight of an ionic resin, including those having a weight average molecular weight of 300,000 and above, as well as those below 300,000, and including those not characterized by silicone macromer portions and particularly those having

silicone macromer portions; and a liquid vehicle comprising 10% to 45% by weight water and 55% to 80% by weight of an organic solvent, preferably selected from the group consisting of ethanol, isopropanol and mixtures thereof.

The level of fluorosurfactant to be used, in general, is given above. It is to be recognized, however, that the particular level of fluorosurfactant which must be used to achieve an improvement in hair spray performance, or to achieve optimum performance, for a particular hair spray composition can vary depending upon a variety of factors, including the particular type of resin chosen and its molecular weight, and level in the composition, the specific flurosurfactant, the level of water and the type and level of volatile organic solvent, and the presence of optional components in the system. In general, higher levels of fluorosurfactant may be needed to achieve a performance benefit as resin molecular weight and/or water levels are reduced. In general, the compositions hereof should contain at least an effective amount of fluorosurfactant to provide an improvement in hair feel for a given level of hair hold performance or for improved spray quality.

Optionally, the hair spray products can contain one or more non-fluorinated surfactant. Generally, if used such non-fluorinated surfactants will be used at a total level of from 0.01% to 2%, preferably from 0.01% to 1.5% and more preferably from 0.01% to 1%, by weight of the composition.

A wide variety of non-fluorinated surfactants can be used, including anionic, cationic, amphoteric, and zwitterionic surfactants.

Anionic surfactants include, for example: alkyl and alkenyl sulfates; alkyl and alkenyl ethoxylated sulfates; (preferably having an average degree of ethoxylation of 1 to 10), succinamate surfactants, such as alkylsulfosuccinamates and dialkyl esters of sulfosuccinic acid; neutralized fatty acid esters of isethionic acid; and alkyl and alkenyl sulfonates, including, for example, olefin sulfonates and beta-alkoxy alkane sulfonates. Preferred are alkyl and alkenyl sulfates and alkyl and alkenyl ethoxylated sulfates such as the sodium and ammonium salts of $C_{12}$-$C_{18}$ sulfates and ethoxylated sulfates with a degree of ethoxylation of from 1 to about 6, preferably from 1 to about 4, e.g., lauryl sulfate and laureth (3.0) sulfate.

Amphoteric surfactants include those which can be broadly described as derivatives of aliphatic secondary and tertiary amines in which the aliphatic radical can be straight chain or branched and wherein one of the aliphatic substituents contains from about 8 to about 18 carbon atoms and one contains an anionic water solubilizing group, e.g., carboxy, sulfonate, sulfate, phosphate, or phosphonate. Examples of compounds falling within this definition are sodium 3-dodecylaminopropionate, N-alkyltaurines such as the one prepared by reacting dodecylamine with sodium isethionate according to the teaching of US-A-2,658,072, N-higher alkyl aspartic acids such as those produced according to the teaching of US-A-2,438,091, and the products sold under the trade name "Miranol" and described in US-A-2,528,378. Others include alkyl, preferably $C_6$-$C_{22}$ and most preferably $C_8$-$C_{12}$, amphoglycinates; alkyl, preferably $C_6$-$C_{22}$ and most preferably $C_8$-$C_{12}$, amphopropionates; and mixtures thereof.

Suitable zwitterionic surfactants for use in the present hair spray products can be exemplified by those which can be broadly described as derivatives of aliphatic quaternary ammonium, phosphonium, and sulfonium compounds, in which the aliphatic radicals can be straight chain or branched, and wherein one of the aliphatic substituents contains from about 8 to 18 carbon atoms and one contains an anionic water-solubilizing group, e.g., carboxy, sulfonate, sulfate, phosphate, or phosphonate. A general formula for these compounds is:

$$\begin{array}{ccccccccc} & & (R^3)_x & & & & & & \\ & & | & & & & & & \\ R^2 & \text{---} & Y^{(+)} & \text{---} & CH_2 & \text{---} & R^4 & \text{---} & Z^{(-)} \end{array}$$

wherein $R^2$ contains an alkyl, alkenyl, or hydroxy alkyl radical of from about 8 to about 18 carbon atoms, from 0 to about 10 ethylene oxide moieties and from 0 to 1 glyceryl moiety; Y is selected from the group consisting of nitrogen, phosphorus, and sulfur atoms; $R^3$ is an alkyl or monohydroxyalkyl group containing 1 to about 3 carbon atoms; x is 1 when Y is sulfur or phosphorus, 1 or 2 when Y is nitrogen; $R^4$ is an alkylene or hydroxyalkylene of from 1 to about 4 carbon atoms and Z is a radical selected from the group consisting of carboxylate, sulfonate, sulfate, phosphonate, and phosphate groups. Classes of zwitterionics include alkyl amino sulfonates, alkyl betaines, and alkyl amido betaines.

Cationic surfactants useful in the hair spray products of the present invention contain amino or quaternary ammonium hydrophilic moieties which are positively charged when dissolved in the aqueous composition of the present invention. Cationic surfactants among those useful herein are disclosed in the following documents: M. C. Publishing Co., McCutcheon's, Detergents & Emulsifiers, (North American edition 1979); Schwartz, et al., Surface Active Agents, Their Chemistry and Technology, New York: Interscience Publishers, 1949; US-A-3,155,591, Hilfer, issued Nov. 3, 1964; US-A-3,929,678, Laughlin, et al., issued Dec. 30, 1975; US-A-3,959,461, Bailey, et al., issued May 25, 1976; and US-A-4,387,090, Bolich, Jr., issued June 7, 1983.

Among the quaternary ammonium-containing cationic surfactants useful herein are those of the general formula:

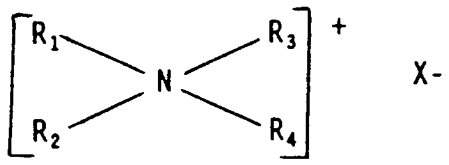

wherein $R_1$ is an aliphatic group of from 1 to 22 carbon atoms, or an aromatic, aryl or alkylaryl group having from 12 to 22 carbon atoms; $R_2$ is an aliphatic group having from 1 to 22 carbon atoms; $R_3$ and $R_4$ are each alkyl groups having from 1 to 3 carbon atoms, and X is an anion selected from halogen, acetate, phosphate, nitrate and alkylsulfate radicals. The aliphatic groups may contain, in addition to carbon and hydrogen atoms, ether linkages, and other groups such as amido groups. Other quaternary ammonium salts useful herein are diquaternary ammonium salts.

Preferred quaternary ammonium salts include dialkyldimethylammonium chlorides, wherein in the alkyl groups have from 12 to 22 carbon atoms and are derived from long-chain fatty acids, such as hydrogenated tallow fatty acid. (Tallow fatty acids give rise to quaternary compounds wherein $R_1$ and $R_2$ have predominately from 16 to 18 carbon atoms.)

Salts of primary, secondary and tertiary fatty amines are also suitable cationic surfactants for use herein. The alkyl groups of such amines preferably have from 12 to 22 carbon atoms, and may be substituted or unsubstituted. Secondary and tertiary amines are preferred, tertiary amines are particularly preferred. Such amines, useful herein, include stearamido propyl dimethyl amine, diethyl amino ethyl stearamide, dimethyl stearamine, dimethyl soyamine, soyamine, myristyl amine, tridecyl amine, ethyl stearylamine, N-tallowpropane diamine, ethoxylated (5 moles E.O.) stearylamine, dihydroxy ethyl stearylamine, and arachidylbehenylamine. Cationic amine surfactants included among those useful in the present invention are disclosed in US-A-4,275,055, Nachtigal, et al., issued June 23, 1981.

Suitable cationic surfactant salts include the halogen, acetate, phosphate, nitrate, citrate, lactate and alkyl sulfate salts.

Nonionic surfactants can also be included. Preferably, the nonionic surfactants have an average HLB (Hydrophile-Lipophile Balance) of less than or equal to about 7.

Methods of determining HLB are well known in the art and any of such methods may be used for HLB determination. A description of the HLB System and methods for HLB determination are described in "The HLB System: a time saving guide to emulsifier selection," ICI Americas Inc.; Wilmington, Delaware; 1976.

Nonionic surfactants include polyethylene oxide condensates of alkyl phenols (preferably $C_6$-$C_{12}$ alkyl, with a degree of ethoxylation of about 1 to about 6), condensation products of ethylene oxide with the reaction product of propylene oxide and ethylene diamine, condensation products of aliphatic alcohols with ethylene oxide, long chain (i.e., typically $C_{12}$-$C_{22}$) tertiary amine oxides, long chain tertiary phosphine oxides, dialkyl sulfoxides containing one long chain alkyl or hydroxy alkyl radical and one short chain (preferably $C_1$-$C_3$) radical, silicone copolyols, and $C_1$-$C_4$ alkanol amides of acids having a $C_8$-$C_{22}$ acyl moiety.

Optionally, the hair spray products of the present invention can contain an effective amount of a non-surface active ionic strength modifier system for reducing the viscosity of the hair spray composition. When used, the ionic strength modifiers will be present in the present products at a level of at least 0.01%, by weight. The upper limit is dependent upon the maximum amount of the ionic strength modifers that can be present such that the hair setting resin remains solubilized or dispersed. As will be understood by those skilled in the art, as the ionic strength of the composition is increased, the resin will eventually fall out of solution, or otherwise no longer remain solubilized or dispersed in the liquid carrier. The upper limit of the ionic strength modifier system level will vary depending upon the particular ionic strength modifiers, liquid vehicle, resin, and other ingredients present in the product. Thus, for example, the maximum amount of the ionic strength modifiers that can be used will tend to be lower for products with liquid vehicles containing less water, compared to compositions with more water. Generally, the compositions will comprise 4%, by weight, or less of the ionic strength modifiers, more generally 2% or less, and typically 1% or less. Preferably, the products hereof will comprise from 0.01% to 0.5%, more preferably from 0.01% to 0.1% by weight, of the ionic strength modifier system.

The ionic strength modifier system comprises a mixture of monomeric cations and anions. The ions of the ionic strength modifier system hereof are non-surface active, i.e. they do not significantly reduce surface tension. For purposes hereof, non-surface active shall mean the ions, which at a 0.5% aqueous solution concentration, reduce surface tension by no more than 5.0 dynes/$cm^2$. Generally, the ions of the ionic strength modifier system hereof will be characterized by having, at maximum, four or less carbon atoms per charge, preferably two or less carbon atoms, in any aliphatic chain or straight or branched chain organic heterochain.

The ionic strength modifier system comprises monomeric ions of the type which are products of acid-base reactions. Thus, basic and acidic ions $OH^-$ and $H^+$ do not constitute part of the ionic strength modifier system hereof, although they may be present in compositions. The ions hereof are incorporated into compositions in a form such that they can exist in the composition as free ions, i.e., in dissociated form. It is not necessary that all of the ions added exist in composition as free ions, but must be at least partially soluble or dissociated in composition. The ionic strength modifiers

can be incorporated into hair styling compositions, for example, by addition of soluble salts, or by addition of mixtures of acids and bases, or by a combination thereof. It is a necessary aspect of the invention that both anions and cations of the ionic strength modifier system be included.

Suitable cations for use include, for example, alkali metals, such as lithium, sodium, and potassium, and alkaline-earth metals, such as magnesium, calcium, and strontium. Preferred of the divalent cations is magnesium. Preferred monovalent metal ions are lithium, sodium, and potassium, particularly sodium and potassium. Suitable means of addition to include, for example, addition as bases, eg., hydroxides, sodium hydroxide and potassium hydroxide, and such as salts that are soluble in the liquid carrier, eg. salts of monomeric anions such as those described below.

Other suitable cations include organic ions, such as quaternary ammonium ions and cationic amines, such as ammonium mono-, di-, and tri-ethanolamines, triethylamine, morpholine, aminomethylpropanol (AMP), aminoethylpropanediol, etc. Ammonium and the amines are preferably provided in the forms of salts, such as hydrochloride salts.

Monomeric anions that can be used include halogen ions, such as chloride, fluoride, bromide, and iodide, particularly chloride, sulfate, ethyl sulfate, methyl sulfate, cyclohexyl sulfamate, thiosulfate, toluene sulfonate, xylene sulfonate, citrate, nitrate, bicarbonate, adipate, succinate, saccharinate, benzoate, lactate, borate, isethionate, tartrate, and other monomeric anions that can exist in dissociated form in the hair styling composition. The anions can be added, for example, in the form of acids or salts which are at least partially soluble in the liquid vehicle, eg., sodium or potassium salts of acetate, citrate, nitrate, chloride, sulfate, etc. Preferably, such salts are entirely soluble in the vehicle.

Hair spray products of the present invention can be dispensed from containers which are aerosol dispensers or pump spray dispensers. Such dispensers, i.e., containers, are well known to those skilled in the art and are commercially available from a variety of manufacturers, including American National Can Corp. and Continental Can Corp.

When hair spray compositions are to be dispensed from a pressurized aerosol container, a propellant which consists of one or more of the conventionally-known aerosol propellants may be used to propel the compositions. A suitable propellant for use can be generally any liquifiable gas conventionally used for aerosol containers.

Suitable propellants for use are volatile hydrocarbon propellants which can include liquified lower hydrocarbons of 3 to 4 carbon atoms such as propane, butane and isobutane. Other suitable propellants are hydrofluorocarbons such as 1,2-difluoroethane (Hydrofluorocarbon 152A) supplied as Dymel 152A (RTM) by DuPont. Other examples of propellants are dimethylether, nitrogen, carbon dioxide, nitrous oxide and atmospheric gas.

The hydrocarbons, particularly isobutane, used singly or admixed with other hydrocarbons are preferred.

The aerosol propellant may be mixed with the compositions and the amount of propellant to be mixed is governed by normal factors well known in the aerosol art. Generally, for liquifiable propellants, the level of propellant is from about 10% to about 60% by weight of the total composition, preferably from about 15% to about 50% by weight of the total composition.

Alternatively, pressurized aerosol dispensers can be used where the propellant is separated from contact with the hair spray composition such as a two compartment can of the type sold under the tradename SEPRO from Americal National Can Corp.

Other suitable aerosol dispensers are those characterized by the propellant being compressed air which can be filled into the dispenser by means of a pump or equivalent device prior to use. Such dispensers are described in US-A-4,077,441, March 7, 1978, Olofsson and US-A-4,850,577, July 25, 1989, TerStege and in U.S. Serial No. 07/839,648, Gosselin et al., filed February 21, 1992. Compressed air aerosol containers suitable for use are also currently marketed by The Procter & Gamble Company under their tradename VIDAL SASSOON AIRSPRAY® (RTM) hair sprays.

Conventional non-aerosol pump spray dispensers, i.e., atomizers, can also be used.

The hair spray products of the present invention can optionally contain conventional hair spray adjuvants. Generally, adjuvants collectively can comprise from 0.05% to 5% by weight and preferably from 0.1% to 3%, by weight. Such conventional optional adjuvants are well known to those skilled in the art and include plasticizers; silicones; emollients; lubricants and penetrants such as various lanolin compounds; protein hydrolysates and other protein derivatives; ethylene adducts and polyoxyethylene cholesterol; dyes, tints and other colorants; sunscreens; and perfume.

The hair spray compositions can be made using conventional formulation and mixing techniques. Preferably, a premix of fluorosurfactant and water is made before addition. If water is not to be used in the composition, a premix of the fluorosurfactant with an organic solvent, such as ethanol, is preferred. Methods of making hair spray compositions are described more specifically in the following examples.

The hair spray compositions are used in conventional ways to provide the hair styling/holding benefits of the present invention. Such method generally involves application of an effective amount of the product to dry and/or slightly damp hair before and/or after the hair is styled. By "effective amount" is meant an amount sufficient to provide the hair hold and style benefits desired considering the length and texture of the hair. Use of the compositions in this manner provides optimum hair holding while exhibiting good sprayability.

The following Examples further illustrate the preferred embodiments within the scope of the present invention.

EXAMPLE I

A hair spray composition which is suitable for use in pump spray dispensers, is prepared as follows:

| Ingredient | Weight % |
|---|---|
| Ethanol, 200 proof | 78.92% |
| Isopropanol | 10.00% |
| Resin[1] | 3.00% |
| KOH (45%)[2] | 0.88% |
| DRO Water[3] | 7.00% |
| Fluorad FC-109® (RTM) (25%)[4] | 0.20% |

[1] 60% t-butyl acrylate/20% acrylic acid/20% silicone macromer weight average mw=10,000, having a weight average molecular weight of about 690,000.
[2] Potassium hydroxide solution, containing 45% potassium hydroxide and 55% water and minors, supplied by Fisher Scientific.
[3] Double reverse osmosis water
[4] Fluorad FC-109® (RTM) supplied by 3M containing 25% potassium fluoroalkyl carboxylates (an anionic fluorosurfactant), 12% propanol, 2% ethanol and 61% water and minors.

The hair spray formulation is prepared by preparing a premix of the resin in isopropanol. The isopropanol premix is added to the ethanol and then neutralized with the potassium hydroxide solution. Then, a premix of the fluorosurfactants and water is prepared and added to the neutralized premix. Other adjuvants, such as fragrances, may then be added. A magnetic or air driven stirrer is used to mix the ingredients until the resin is dissolved, typically about 1 to 2 hours.

EXAMPLE II

A hair spray composition, which is suitable for use in pump spray dispensers, is prepared as follows:

| Ingredient | Weight % |
|---|---|
| Ethanol, 200 proof | 79.05% |
| Resin[1] | 2.60% |
| KOH (45%) | 0.75% |
| DRO Water | 7.00% |
| Fluorad FC-120® (RTM) (25%)[2] | 0.20% |

[1] 60% t-butyl acrylate/20% acrylic acid/20% silicone macromer weight average mw=10,000, having a weight average molecular weight of about 800,000.
[2] Fluorad FC-120® (RTM) supplied by 3M, having anionic character and containing 25% mixed ammonium perfluoroalkyl sulfonates, 37.5% ethanol and 37.5% water and minors.

The hair spray formulation is prepared by preparing a premix of the resin in ethanol. The ethanol premix is then neutralized with the potassium hydroxide solution. Then, a premix of the fluorosurfactants and water is prepared and

added to the neutralized premix. Other adjuvants, such as fragrances, may then be added. A magnetic or air driven stirrer is used to mix the ingredients until the resin is dissolved, typically about l to 2 hours.

EXAMPLE III

A hair spray composition which is suitable for use in pump spray dispensers, is prepared as follows:

| Ingredient | Weight % |
|---|---|
| Ethanol, 200 proof | 79.10% |
| Isopropanol | 10.40% |
| Resin[1] | 2.60% |
| KOH (45%) | 0.75% |
| DRO Water | 7.00% |
| Fluorad FC-120® (RTM) (25%) | 0.10% |
| Zonyl FSK (RTM) (47%) | 0.05% |

[1] 60% t-butyl acrylate /20% acrylic acid/20% silicone macromer weight average mw=10,000, having a weight average molecular weight of about 800,000.

This composition is prepared as in Example I

EXAMPLE IV

A hair spray composition which is suitable for use in pump spray dispensers, is prepared as follows:

| Ingredient | Weight % |
|---|---|
| Ethanol, 200 proof | 78.86% |
| Isopropanol | 10.40% |
| Resin[1] | 2.60% |
| KOH (45%) | 0.69% |
| DRO Water | 7.00% |
| Fluorad FC-120® (RTM) (25%) | 0.40% |
| Zonyl FSK® (RTM) (47%) | 0.05% |

[1] 60% t-butyl acrylate /20% acrylic acid/20% silicone macromer weight average mw=10,000, having a weight average molecular weight of about 1,700,000.

This composition is prepared as in Example I

EXAMPLE V

A hair spray composition which is suitable for use in pump spray dispensers, is prepared as follows:

| Ingredient | Weight % |
|---|---|
| Ethanol, 200 proof | 71.18% |
| Isopropanol | 9.52% |
| Resin[1] | 2.38% |
| KOH (45%) | 0.59% |
| DRO Water | 16.19% |
| Zonyl TBS® (RTM) (35%)[2] | 0.14% |

[1] 60% t-butyl acrylate/20% acrylic acid/20% silicone macromer weight average mw=10,000, having a weight average molecular weight of about 800,000.

[2] Zonyl TBS® (RTM) supplied by DuPont having an anionic character and containing 30-35% perfluoroalkyl sulfonic acid, ammonium salt, 2.4% acetic acid and 65%-70% water and minors.

This composition is prepared as in Example I.

EXAMPLE VI

A hair spray composition which is suitable for use in pump spray dispensers, is prepared as follows:

| Ingredient | Weight % |
|---|---|
| Ethanol, 200 proof | 80.00% |
| Resin[1] | 2.38% |
| KOH (45%) | 0.59% |
| DRO Water | 16.94% |
| Zonyl FSK® (RTM) (47%) | 0.10% |

[1] 60% t-butyl acrylate/20% acrylic acid/20% silicone macromer weight average mw=10,000, having a weight average molecular weight of about 800,000.

This composition is prepared as in Example II.

EXAMPLE VII

A hair spray composition which is suitable for use in pump spray dispensers, is prepared as follows:

| Ingredient | Weight % |
|---|---|
| Ethanol, 200 proof | 71.20% |
| Isopropanol | 9.52% |
| Resin[1] | 2.38% |
| KOH (45%) | 0.59% |
| DRO Water | 16.19% |
| Zonyl FSK® (RTM) (47%) | 0.05% |
| Zonyl TBS® (RTM) (35%) | 0.07% |

[1] 75% ethyl methacrylate/20% acrylic acid/5% silicone macromer weight average mw=10,000, having a weight average molecular weight of about 1,000,000.

This composition is prepared as in Example I.

EXAMPLE VIII

A hair spray composition which is suitable for use in pump spray dispensers, is prepared as follows:

| Ingredient | Weight % |
|---|---|
| Ethanol, 200 proof | 78.99% |
| Isopropanol | 10.40% |
| Resin[1] | 2.60% |
| AMP[2] | 0.16% |
| KOH (45%) | 0.63% |
| DRO water | 7.00% |
| Fluorad FC-120® (RTM) (25%) | 0.10% |
| Zonyl FSK® (RTM) (47%) | 0.05% |
| Zonyl TBS® (RTM) (35%) | 0.07% |

[1] 60% t-butyl acrylate/20% acrylic acid/20% silicone macromer weight average mw=10,000, having a weight average molecular weight of about 860,000.
[2] Aminomethyl propanol.

This composition is prepared as in Example I.

EXAMPLE IX

A hair spray composition which is suitable for use in pump spray dispensers, is prepared as follows:

| Ingredient | Weight % |
|---|---|
| Ethanol, 200 proof | 80.00% |
| Resin[1] | 2.60% |
| KOH (45%) | 0.44% |
| DRO Water | 16.00% |
| Fluorad FC-120® (RTM) (25%) | 0.76% |
| Fragrance | 0.20% |

[1] 60% t-butyl acrylate/20% acrylic acid/20% silicone macromer weight average mw=10,000, having a weight average moleculer weight of about 2,000,000.

This composition is prepared as in Example I except that the fluorosurfactant is premixed with ethanol instead of isopropanol.

EXAMPLE X

A hair spray composition which is suitable for use in pump spray dispensers, is prepared as follows:

| Ingredient | Weight % |
|---|---|
| Ethanol, 200 proof | 70.73% |
| Isoproponol | 8.80% |
| Resin[1] | 2.20% |
| KOH (45%) | 0.37% |
| DRO Water | 17.00% |
| $AE_3S$ (28%)[2] | 0.30% |
| Fluorad FC-100® (RTM) (25%) | 0.40% |
| Fragrance | 0.20% |

[1] 60% t-butyl acrylate/20% acrylic acid/20% silicone macromer weight average mw=10,000, having a weight average molecular weight of about 800,000.
[2] Ammonium laureth sulfate solution containing 28% ammonium laureth sulfate 3 moles of ethoxyl molecule and 72% water and minors, supplied by Stepan.

This composition is prepared as in Example 1.

EXAMPLE XI

A hair spray composition which is suitable for use in pump spray dispensers, is prepared as follows:

| Ingredient | Weight % |
|---|---|
| Ethanol, 200 proof | 79.00% |
| Resin[1] | 4.00% |
| KOH (45%) | 0.84% |
| DRO Water | 15.86% |
| Aerosol OT | 0.25% |
| Fluorad FC-120® (RTM) | 0.20% |

[1] 60% t-butyl acrylate/20% acrylic acid/20% silicone macromer weight average mw=10,000, having a weight average molecular weight of about 500,000.

[2] Aerosol OT® (RTM), a sodium diioctyl sulfosuccinate surfactant available from American Cyanamid as a 75% active solution in water and ethanol.

This composition is prepared as in Example II.

EXAMPLES XII - XV

The following hairspray compositions are prepared, and can be dispensed in a nonaerosol, pump spray container in compressed air, pump spray containers, or in combination with a chemical propellant in a conventional aerosol container.

| Ingredient | Example # (weight %) | | | |
|---|---|---|---|---|
| | XII | XIII | XIV | XV |
| Ethanol, 200 proof | 78.76 | 78.00 | 78.00 | 78.76 |
| Water | 15.55 | 14.15 | 14.62 | 16.14 |
| Fluorad FC-120 (25% active) | 0.14 | 0.14 | 0.14 | 0.14 |
| Amphomer®[1] (RTM) | 4.00 | 5.20 | 4.50 | 3.50 |
| Aminomethylpropanol (AMP) | 0.66 | 0.86 | 0.74 | 0.58 |
| Dimethicone Copolyol | 0.50 | 0.10 | 0.25 | 0.24 |
| Cyclomethicone | 0.24 | 1.00 | 1.00 | 0.44 |
| Fragrance and other minors | 0.16 | 0.56 | 0.75 | 0.15 |

[1] Amphomer® (RTM)-octoylacrylamide/acrylates/butylaminoethyl methacrylate copolymer, available from National Starch and Chemical Co. (Bridgewater, NJ, USA), about 70,000 weight average MW.

All of the above compositions when sprayed on to hair exhibit good sprayability, hair feel, and hold.

In the above examples and the compositions hereof utilizing silicone macromer-grafted styling resins, it can be desirable to purify the styling resin by removing unreacted silicone-containing monomer and silicone macromer-grafted polymer with viscosities at 25°C of about 10,000,000 centistokes and less. This can be done, for the example, by hexane extraction. After drying the resin from its reaction solvent, hexane extraction of the reaction product can be performed by adding an excess of hexane to the reaction product and heating to near the Tg of the non-silicone portion of the

polymer. The mixture is held at this temperature with stirring for about 30 minutes and cooled to room temperature. The hexane is removed by vacuum suction. Two more hexane extraction cycles are preferably conducted in the same manner as above. After the third cycle, residual hexane remaining with the product is removed by distillation and vacuum drying.

## Claims

1. A low volatile organic solvent hair spray product comprising a hairspray composition and a spray dispenser means for containing and spraying said hairspray composition, said hairspray composition being contained in said spray dispenser means, wherein said hairspray composition characterized in that it comprises:

    (a) from 0.01% to 2%, by weight, of an ionic fluorosurfactant;
    (b) from 0.1% to 15%, by weight, of an anionic and/or amphoteric hair setting resin; and
    (c) a liquid vehicle comprising from 10% to 70%, by weight of the composition, water and from 30% to 80%, by weight of the

    composition, of monohydric alcohol.

2. A hair spray product as in Claim 1, wherein said composition comprises from 11% to 60% water, preferably from 13% to 50% water, more preferably from 14% to 50% water.

3. A hairspray product as in Claim 1 or 2, wherein said resin has a weight average molecular weight of at least 70,000, preferably at least 100,000, more preferably from 100,000 to 1,000,000, most preferably from 125,000 to 200,000.

4. A hairspray product as in any of claims 1 to 3, wherein said resin comprises from 2% to 75%, by weight, ionic monomer and from 25% to 98%, by weight, nonionic monomer, and wherein said resin contains at least 0.5%, by weight of silicone-macromer containing monomer.

5. A hairspray product as in Claim 4, wherein said ionic monomer is acrylic acid, methacrylic acid, dimethylaminoethyl methacrylate, quaternized dimethylaminoethyl, methacrylate, maleic acid, half esters of maleic anhydride, crotonic acid, itaconic acid, diallyldimethyl ammonium chloride, vinyl pyridine, vinyl imidazole, styrene sulfonate, or a mixture thereof, said nonionic monomer is selected from the group consisting of acrylic acid esters of $C_1$-$C_{24}$ alcohols, methacrylic acid esters of $C_1$-$C_{24}$ alcohols, styrene, polystyrene macromer, vinyl acetate, vinyl chloride, vinyl propionate, vinylidene chloride, alphamethylstyrene, t-butylstyrene, butadiene, cyclohexadiene, ethylene, propylene, vinyl toluene, and mixtures thereof, and said silicone-macromer containing monomer has the formula:

    $$X(Y)_nSi(R)_{3-m}Z_m$$

    wherein X is a vinyl group; Y is a divalent linking group; R is a hydrogen, hydroxyl, lower alkyl, aryl, alkaryl, alkylamino, or alkoxy; Z is a monovalent siloxane polymeric moiety having a number average molecular weight of at least 500; n is 0 or 1; and m is an integer from 1 to 3.

6. A liquid hair spray product as in any of Claims 4 or 5, wherein said silicone macromer-containing monomer is:

    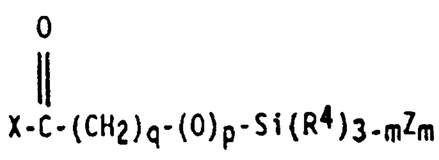

    wherein m is 1, 2, or 3, p is 0 or 1, q is an integer from 2 to 6, X is

    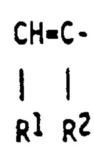

    $R^1$ is -H or COOH, $R^2$ is -H, $-CH_3$, or $CH_2$ COOH, $R^4$ is alkyl, alkoxy, alkylamino, aryl, alkaryl, hydrogen or hydroxyl,

and Z is

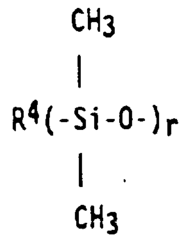

wherein r is an integer at least 5.

7. A hair spray product according to any of Claims 1-6 wherein the ionic fluorosurfactant is selected from:

1) fluorinated alkyl quaternary ammonium iodides;
2) mono- and bis-perfluoroalkyl phosphates, mono- and bis-fluoroalkyl phosphate, complexed with aliphatic quaternary methosulfates; salts of perfluoroalkyl sulfonic acid; telomer phosphate diethanolamine salts; amine perfluoroalkyl sulfonates; ammonium perfluoroalkyl sulfonates; potassium perfluoroalkyl sulfonates; fluorinated alkyl carboxylates;
3) fluorinated alkyl sulfonates; and
4) mixtures thereof.

8. A hairspray product as in any of Claims 1 to 7, wherein said means for containing and spraying said composition is an aerosol spray container.

9. A hair spray product as in Claim 8, further comprising an aerosol propellant disposed within said container.

10. A hair spray product as in Claim 8, wherein said aerosol spray container is a pump spray container, wherein air is utilized as a propellant.

11. A hair spray product as in any of Claims 1 to 7 wherein said means for containing and spraying said composition is a nonaerosol pump spray container.

12. A hairspray product as in any of Claims 1-11, wherein said fluorosurfactant is a perfluoroalkyl sulfonate or a fluorinated alkyl quaternary ammonium chloride, or a mixture thereof.

13. A method for providing hair setting benefits to the hair, comprising spraying an effective amount of the compositions contained in the hair spray product of any of Claims 1-12 to hair.

**Patentansprüche**

1. Ein, ein gering flüchtiges organisches Lösungsmittel enthaltendes Haarsprayprodukt, umfassend eine Haarsprayzusammensetzung und ein Sprüh-Abgabemittel, welches die genannte Haarsprayzusammensetzung enthält und womit die genannte Haarsprayzusammensetzung versprüht wird, welche Haarsprayzusammensetzung in dem genannten Sprüh-Abgabemittel enthalten ist, wobei die genannte Haarsprayzusammensetzung dadurch gekennzeichnet ist, daß sie

(a) 0,01 Gew.-% bis 2 Gew.-% eines ionischen fluorhaltigen grenzflächenaktiven Mittels;
(b) 0,1 Gew.-% bis 15 Gew.-% eines anionischen und/oder amphoteren haarfestigenden Harzes; und
(c) einen flüssigen Träger enthält, welcher 10 Gew.-% bis 70 Gew.-% der Zusammensetzung an Wasser und 30 Gew.-% bis 80 Gew.-% der Zusammensetzung an einwertigem Alkohol enthält.

2. Haarsprayprodukt nach Anspruch 1, wobei die genannte Zusammensetzung 11% bis 60% Wasser, vorzugsweise 13% bis 50% Wasser, stärker bevorzugt 14% bis 50% Wasser umfaßt.

3. Haarsprayprodukt nach Anspruch 1 oder 2, wobei das genannte Harz ein Gewichtsmittel-Molekulargewicht von mindestens 70 000, vorzugsweise von mindestens 100 000, stärker bevorzugt von 100 000 bis 1 000 000, am stärksten bevorzugt von 125 000 bis 200 000 aufweist.

4. Haarsprayprodukt nach einem der Ansprüche 1 bis 3, wobei das genannte Harz 2 Gew.-% bis 75 Gew.-% an ionischem Monomer und 25 Gew.-% bis 98 Gew.-% an nichtionischem Monomer umfaßt und wobei das genannte Harz mindestens 0,5 Gew.-% von einem ein Siliconmakromer enthaltenden Monomer aufweist.

5. Haarsprayprodukt nach Anspruch 4, wobei das genannte ionische Monomer von Acrylsäure, Methacrylsäure, Dimethylaminoethylmethacrylat, quaterniertes Dimethylaminoethyl, Methacrylat, Maleinsäure, Halbestern von Maleinsäureanhydrid, Crotonsäure, Itaconsäure, Diallyldimethylammoniumchlorid, Vinylpyridin, Vinylimidazol, Styrolsulfonat oder einam Gemisch hievon gebildet wird, wobei das genannte nichtionische Monomer von der aus Acrylsäureestern von $C_1$-$C_{24}$-Alkoholen, Methacrylsäureestern von $C_1$-$C_{24}$-Alkoholen, Styrol, Polystyrolmakromer, Vinylacetat, Vinylchlorid, Vinylpropionat, Vinylidenchlorid, $\alpha$-Methylstyrol, t-Butylstyrol, Butadien, Cyclohexadien, Ethylen, Propylen, Vinyltoluol und Gemischen hievon bestehenden Gruppe ausgewählt ist, und das genannte ein Siliconmakromer enthaltende Monomer die Formel

$$X(Y)_nSi(R)_{3-m}Z_m$$

besitzt, worin X eine Vinylgruppe darstellt, Y eine zweiwertige verbindende Gruppe ist; R für Wasserstoff, Hydroxyl, Niederalkyl, Aryl, Alkaryl, Alkylamino oder Alkoxy steht; Z einen einwertigen polymeren Siloxanrest mit einem ZahlenmittelMolekulargewicht von mindestens 500 darstellt, n für 0 oder 1 steht und m eine ganze Zahl von 1 bis 3 bedeutet.

6. Flüssiges Haarsprayprodukt nach einem der Ansprüche 4 oder 5, wobei das genannte ein Silikonmakromer enthaltende Monomer

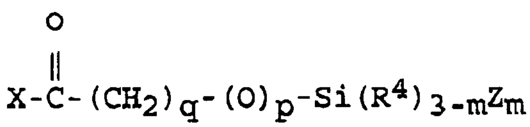

ist, worin m für 1, 2 oder 3 steht, p 0 oder 1 ist, q eine ganze Zahl von 2 bis 6 bedeutet, X für

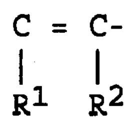

steht, $R^1$ -H oder -COOH bedeutet, $R^2$ -H, $-CH_3$, oder $CH_2COOH$ darstellt, $R^4$ Alkyl, Alkoxy, Alkylamino, Aryl, Alkaryl, Wasserstoff oder Hydroxyl ist, und Z für

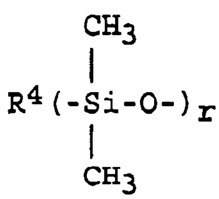

steht, worin r eine ganze Zahl von mindestens 5 ist.

7. Haarsprayprodukt nach einem der Ansprüche 1 bis 6, wobei das ionische fluorhaltige grenzflächenaktive Mittel unter

1) fluorierten Alkyl-quaternären Ammoniumiodiden;
2) Mono- und Bisperfluoralkylphosphaten, Mono- und Bisperfluornlkylphosphat, welche mit quaternären aliphatischen Methosulfaten komplexiert sind; Salzen der Perfluoralkylsulfonsäure; telomeren Phosphatdiethanolaminsalzen; Aminperfluoralkylsulfonaten; Ammoniumperfluoralkylsulfonaten; Kaliumperfluoralkylsulfonaten; fluorierten Alkylcarboxylaten;
3) fluorierten Alkylsulfonaten; und
4) Gemischen hievon

ausgewählt ist.

8. Haarsprayprodukt nach einem der Ansprüche 1 bis 7, wobei das genannte Mittel, welches die genannte Zusammensetzung enthält und wodurch diese versprüht wird, ein Aerosol-Sprühbehälter ist.

9. Haarsprayprodukt nach Anspruch 8, welches ferner ein im genannten Behälter enthaltenes Aerosoltreibmittel umfaßt.

10. Haarsprayprodukt nach Anspruch 8, wobei der genannte Aerosol-Sprühbehälter ein Pumpsprühbehälter ist, worin Luft als Treibmittel verwendet wird.

11. Haarsprayprodukt nach einem der Ansprüche 1 bis 7, wobei das genannte Mittel, welches die genannte Zusammensetzung enthält und wodurch diese versprüht wird, ein Aerosol-freier Pumpsprühbehälter ist.

12. Haarsprayprodukt nach einem der Ansprüche 1 bis 11, wobei das genannte fluorhaltige grenzflächenaktive Mittel ein Perfluoralkylsulfonat oder ein fluoriertes Alkyl-quaternäres Ammoniumchlorid oder ein Gemisch hievon ist.

13. Verfahren zur Gewährleistung von Haarfestigungsvorteilen für das Haar, umfassend das Aufsprühen einer wirksamen Mengen, der im Haarsprayprodukt enthaltenen Zusammensetzung nach einem der Ansprüche 1 bis 12 auf das Haar.

## Revendications

1. Produit d'atomisation pour les cheveux à base de solvant organique faiblement volatil, comprenant une composition d'atomisation pour les cheveux et un dispositif atomiseur destiné à contenir et à atomiser ladite composition d'atomisation pour les cheveux, ladite composition d'atomisation pour les cheveux étant contenue dans ledit dispositif atomiseur, dans lequel ladite composition d'atomisation pour les cheveux est caractérisée par le fait qu'elle comprend:

   (a) de 0,01% à 2%, en poids, d'un tensioactif fluoré ionique;
   (b) de 0,1% à 15%, en poids, d'une résine fixante pour les cheveux, anionique et/ou amphotère; et
   (c) un véhicule liquide comprenant de 10% à 70%, en poids de la composition, d'eau et de 30% à 80%, en poids de la composition, de monoalcool.

2. Produit d'atomisation pour les cheveux selon la revendication 1, dans lequel ladite composition comprend de 11% à 60% d'eau, de préférence de 13% à 50% d'eau, mieux encore de 14% à 50% d'eau.

3. Produit d'atomisation pour les cheveux selon la revendication 1 ou 2, dans lequel ladite résine possède une masse moléculaire moyenne en poids d'au moins 70 000, de préférence d'au moins 100 000, mieux encore de 100 000 à 1 000 000, tout particulièrement de 125 000 à 200 000.

4. Produit d'atomisation pour les cheveux selon l'une quelconque des revendications 1 à 3, dans lequel ladite résine comprend de 2% à 75%, en poids, de monomère ionique et de 25% à 98%, en poids, de monomère non ionique, et dans lequel ladite résine contient au moins 0,5%, en poids, de monomère contenant un macromère de silicone.

5. Produit d'atomisation pour les cheveux selon la revendication 4, dans lequel ledit monomère ionique est l'acide acrylique, l'acide méthacrylique, le méthacrylate de diméthylaminoéthyle, le méthacrylate de diméthylaminoéthyle quaternisé, l'acide maléique, les semi-esters d'anhydride maléique, l'acide crotonique, l'acide itaconique, le chlorure de diallyldiméthylammonium, la vinylpyridine, le vinylimidazole, le styrènesulfonate, ou un mélange de ceux-ci, ledit monomère non ionique est choisi dans le groupe constitué par les esters d'acide acrylique et d'alcools en $C_1$-$C_{24}$, les esters d'acide méthacrylique et d'alcools en $C_1$-$C_{24}$, le styrène, un macromère de polystyrène, l'acétate de vinyle, le chlorure de vinyle, le propionate de vinyle, le chlorure de vinylidène, l'alpha-méthylstyrène, le t-butylstyrène, le butadiène, le cyclohexadiène, l'éthylène, le propylène, le vinyltoluène, et des mélanges de ceux-ci, et ledit monomère contenant un macromère de silicone a pour formule:

$$X(Y)_nSi(R)_{3-m}Z_m$$

dans laquelle X est un groupe vinyle; Y est un groupe de liaison divalent; R est un atome d'hydrogène ou un groupe hydroxyle, alkyle inférieur, aryle, alkylaryle, alkylamino ou alcoxy; Z est un groupement polymère siloxane monovalent ayant une masse moléculaire moyenne en nombre d'au moins 500; n vaut 0 ou 1; et m est un nombre entier de 1 à 3.

6. Produit liquide d'atomisation pour les cheveux selon l'une quelconque des revendications 4 ou 5, dans lequel ledit monomère contenant un macromère de silicone est:

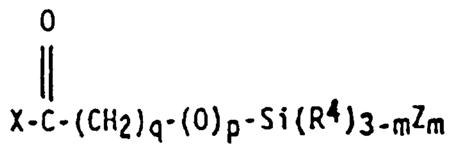

où m vaut 1, 2 ou 3, p vaut 0 ou 1, q est un nombre entier de 2 à 6, X est

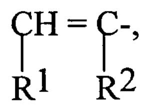

$R^1$ est -H ou -COOH, $R^2$ est -H, $-CH_3$ ou $CH_2COOH$, $R^4$ est un groupe alkyle, alcoxy, alkylamino, aryle, alkylaryle, un atome d'hydrogène ou un groupe hydroxyle, et Z est

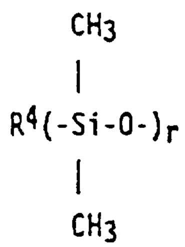

où r est un nombre entier au moins égal à 5.

7. Produit d'atomisation pour les cheveux selon l'une quelconque des revendications 1-6, dans lequel le tensioactif fluoré ionique est choisi parmi:

1) les iodures d'alkylammonium quaternaire fluorés;
2) les phosphates de mono- et bis-perfluoroalkyle, les phosphates de mono- et bis-fluoroalkyle complexés avec des méthylsulfates quaternaires aliphatiques; les sels d'acide perfluoroalkylsulfonique; les sels télomères phosphate-diéthanolamine; les perfluoroalkylsulfonates d'amine; les perfluoroalkylsulfonates d'ammonium; les perfluoroalkylsulfonates de potassium; les alkylcarboxylates fluorés;
3) les alkylsulfonates fluorés; et
4) les mélanges de ceux-ci.

8. Produit d'atomisation pour les cheveux selon l'une quelconque des revendications 1 à 7, dans lequel ledit dispositif destiné à contenir et à atomiser ladite composition est un récipient atomiseur d'aérosol.

9. Produit d'atomisation pour les cheveux selon la revendication 8, comprenant aussi un propulseur d'aérosol placé à l'intérieur dudit récipient.

10. Produit d'atomisation pour les cheveux selon la revendication 8, dans lequel ledit récipient atomiseur d'aérosol est un récipient atomiseur à pompe, dans lequel l'air est utilisé comme propulseur.

11. Produit d'atomisation pour les cheveux selon l'une quelconque des revendications 1 à 7, dans lequel ledit dispositif destiné à contenir et à atomiser ladite composition est un récipient atomiseur à pompe non aérosol.

**12.** Produit d'atomisation pour les cheveux selon l'une quelconque des revendications 1-11, dans lequel ledit tensioactif fluoré est un perfluoroalkylsulfonate ou un chlorure d'alkylammonium quaternaire fluoré, ou un mélange de ceux-ci.

**13.** Procédé pour apporter des avantages de fixation des cheveux à la chevelure, comprenant l'atomisation sur la chevelure d'une quantité efficace de la composition contenue dans le produit d'atomisation pour les cheveux selon l'une quelconque des revendications 1-12.